(19) Europäisches Patentamt
European Patent Office
Office européen des brevets

(11) **EP 4 115 805 A1**

(12) **EUROPÄISCHE PATENTANMELDUNG**

(43) Veröffentlichungstag:
**11.01.2023 Patentblatt 2023/02**

(21) Anmeldenummer: **22183460.9**

(22) Anmeldetag: **06.07.2022**

(51) Internationale Patentklassifikation (IPC):
**A61B 5/11** (2006.01)    **G06V 10/44** (2022.01)
**A61B 5/00** (2006.01)

(52) Gemeinsame Patentklassifikation (CPC):
**A61B 5/112; A61B 5/0077; A61B 5/1128;**
**A61B 5/4082; A61B 5/4836; A61B 5/486;**
**A61B 5/6898; A61B 5/7264; A61B 5/7282;**
**A61B 5/7415; A61B 5/7425; A61B 5/7455;**
**G06V 10/44;** A61B 5/7275; A61B 5/745;    (Forts.)

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**
Benannte Erstreckungsstaaten:
**BA ME**
Benannte Validierungsstaaten:
**KH MA MD TN**

(30) Priorität: **07.07.2021 DE 102021117544**

(71) Anmelder: **Frederik Hauke & Felix Möller digitale**
**Medizintechnik GbR**
**40589 Düsseldorf (DE)**

(72) Erfinder: **Hauke, Frederik**
**50589 Düsseldorf (DE)**

(74) Vertreter: **Bausch, Thomas**
**Weg zur Schanze 35**
**58313 Herdecke (DE)**

(54) **VERFAHREN UND VORICHTUNG ZUR UNTERSTÜTZUNG EINER GEHBEWEGUNG**

(57)    Verfahren und Vorrichtung, im einfachsten Fall unter Verwendung eines handelsüblichen Smartphones mit einer eigens hierfür geeigneten App. Verfahren und Vorrichtung dienen zur Unterstützung einer Gehbewegung einer Person mit einer Bewegungsstörung, das Smartphone aufweisend ein Display, eine rückseitige Farbkamera, die für AR-Anwendungen kalibriert ist, und Sensoren für die Position des Gerätes im dreidimensionalen Raum und die Geometrie der Umgebung integriert hat, eine Schnittstelle für ein AR-Toolkit sowie ein AR-Toolkit, Sensoren für Sensordaten aus einer Inertial Measurement Unit, umfassend die folgenden Schritte: anfängliche Bestimmung von Position und Ausrichtung des Smartphones, anfängliche Prüfung, ob die Ausrichtung der Kamera und das Sichtfeld einen Blick auf die Füße des Nutzers zulässt, Mustererkennung zur Erkennung der Füße und deren genauer Position für jeden Zeitschritt, Definition der Bounding-Boxen für die Füße und deren Koordinaten einschließlich der Höhenkoordinaten für jeden Zeitschritt, Zuordnung der Position der Bounding-Boxen zum rechten und linken Fuß für jeden Zeitschritt, Analyse der Zuverlässigkeit der Positionsdaten für jeden Zeitschritt, Übergabe der Bildschirmkoordinaten der Bounding-Boxen an das AR-Toolkit, für jeden Zeitschritt, Anfertigung eines Abbildes des Raumes durch die rückseitige Kamera und Zuordnung jedes Bildpunktes einem Punkt des abgebildeten Raumes durch das AR-Toolkit für jeden Zeitschritt, Anfertigung einer zeitlichen Serie von Raumpunkten der Bounding-Boxen für jeden Zeitschritt, Klassifikation der Detektion der Füße für jeden Zeitschritt, Reduktion des Messrauschens für jeden Zeitschritt, Bestimmung von Schrittfrequenz und Schrittlänge für jeden Zeitschritt, Generierung von Prädiktionen über die zukünftigen Positionen der Füße für jeden Zeitschritt, Feststellung des "Freezing of Gait", bei Erreichen von Schrittlänge und Schrittfrequenz Null für Prädiktion, Ausgabe von Stimulation an den Nutzer, Erwarten der Reaktion des Nutzers.

EP 4 115 805 A1

(52) Gemeinsame Patentklassifikation (CPC): (Forts.)
A61B 2560/0223

**Beschreibung**

Die Erfindung betrifft eine Vorrichtung und ein Verfahren zur Hilfe für Patienten bei Parkinson-Erkrankungen. Die Einschränkungen und Probleme beim Auftreten einer Parkinson-Erkrankung sind gut bekannt. Problematisch ist vor allem das sogenannte "Freezing of Gait", im Folgenden als FOG abgekürzt, was bedeutet, dass der Patient plötzlich keinen Schritt mehr vor den anderen setzen kann, er friert praktisch ein und kann aktiv willentlich aus diesem Zustand nicht herausfinden.

Es sind auch eine Reihe von Technologien entwickelt worden, um dem zu begegnen, in den meisten Fällen ist hierfür die Anwesenheit eines Therapeuten erforderlich. Außerdem ist eine Vielzahl von Geräten im Einsatz, um den Patienten zu stabilisieren, damit er nicht das Gleichgewicht verliert. Es wird auch versucht, den Patienten zu trainieren.

Die DE 10 2014 215 487 A1 beschreibt ein Verfahren zur Unterstützung einer Bewegung einer Person mit einer Bewegungsstörung, weiter auf eine Vorrichtung und ein computergestütztes Signalsystem für eine Person mit einer Bewegungsstörung, wie sie beispielsweise für Parkinson typisch ist. Hierbei wird dem Patienten ein Signal mittels eines durch das Signal erzeugten Reizes übermittelt, wobei der Reiz als visueller, akustischer oder haptischer Reiz erfolgen kann. Beispielsweise kann es sich dabei um eine ins Sichtfeld der Person eingeblendete geometrische Figur handeln.

Die Schrift Mazilu, S. et. Al: GaitAssist: A Daily-Life Support and Training System for Parkinson's Disease Patients with Freezing of Gait, CHI 2014, One of a CHInd, Toronto, ON, Canada; Session Interactive Technologies for Rehabilitation; S 2531-2540, beschreibt ein tragbares System zur Unterstützung beim FOG, bei dem akustische Signale zum Trainieren von FOG-Situationen verwendet werden.

Allerdings muss großer Aufwand getrieben werden, um das Signal zu erzeugen, etwa indem stresserzeugende Umweltfaktoren ermittelt werden, die mit einem zuvor ermittelten patientenspezifischen Grenzwert für den Stress, bei dem die Bewegungsstörung auftritt, verglichen werden müssen. Auch müssen Bewegungs- und Positionsdaten erfasst werden, was mithilfe von tragbaren Geräten geschehen kann, die mit Displays und Kameras ausgestattet sind. Mit deren Hilfe können dann beispielsweise visuelle Reize bzw. Cues in den Raum oder auf den Boden projiziert werden. Auch ist entsprechende Sensorik und zugehöriger Auswertungsaufwand erforderlich, um den FOG zutreffend erkennen und mit dem mutmaßlich stressauslösenden Ereignis korrelieren zu können.

Beispielsweise werden in das Sichtfeld der Person visuelle Reize eingeblendet, etwa eine Mehrzahl virtueller dreidimensionaler Balken quer zur beabsichtigten Bewegungsrichtung der Person. Durch Größe und Anordnung in der virtuellen Darstellung der Balken wird ein räumlicher Effekt im Sinne der Erweiterten Realität bzw. Augmented Reality (im Folgenden als AR bezeichnet) erzielt und so die bewegungsfördernde Wirkung des visuellen Reizes vorteilhaft verstärkt.

Die bekannten Technologien dienen meist der Therapie und wirken nur in bekannten Therapieumgebungen. Wünschenswert sind aber alltagstaugliche Technologien, die es dem Patienten ermöglichen, ein normales Leben zu führen und nur im Bedarfsfall Unterstützung zu benötigen und diese Unterstützung auch ohne Umstände abfordern zu können. Auch soll dem Patienten nicht zugemutet werden, stets aufwändige und auffällige Hilfsmittel mitführen zu müssen.

Die Erfindung löst die Aufgabe mittels eines Verfahrens und einer Vorrichtung, im einfachsten Fall unter Verwendung eines handelsüblichen Smartphones mit einer eigens hierfür geeigneten App. Verfahren und Vorrichtung dienen zur Unterstützung einer Gehbewegung einer Person mit einer Bewegungsstörung, das Smartphone aufweisend

- ein Display,

- eine rückseitige Farbkamera, die für AR-Anwendungen kalibriert ist, und Sensoren für die Position des Gerätes im dreidimensionalen Raum und die Geometrie der Umgebung integriert hat,

- eine Schnittstelle für ein AR-Toolkit sowie ein AR-Toolkit,

- Sensoren für Sensordaten aus einer "inertial measurement unit", im Folgenden als IMU bezeichnet,

umfassend die folgenden Schritte:

- anfängliche Bestimmung von Position und Ausrichtung des Smartphones,

- anfängliche Prüfung, ob die Ausrichtung der Kamera und das Sichtfeld einen Blick auf die Füße des Nutzers zulässt,

- Mustererkennung zur Erkennung der Füße und deren genauer Position für jeden Zeitschritt,

- Definition der Bounding-Boxen für die Füße und deren Koordinaten einschließlich der Höhenkoordinaten für jeden Zeitschritt,

- Zuordnung der Position der Bounding-Boxen zum rechten und linken Fuß für jeden Zeitschritt,

- Analyse der Zuverlässigkeit der Positionsdaten für jeden Zeitschritt,

- Übergabe der Bildschirmkoordinaten der Bounding-Boxen an das AR-Toolkit, für jeden Zeitschritt,

- Anfertigung eines Abbildes des Raumes durch die rückseitige Kamera und Zuordnung jedes Bildpunktes einem Punkt des abgebildeten Raumes durch das AR-Toolkit für jeden Zeitschritt,

- Anfertigung einer zeitlichen Serie von Raumpunkten der Bounding-Boxen für jeden Zeitschritt,

- Klassifikation der Detektion der Füße für jeden Zeitschritt,

- Reduktion des Messrauschens für jeden Zeitschritt,

- Bestimmung von Schrittfrequenz und Schrittlänge für jeden Zeitschritt,

- Generierung von Prädiktionen über die zukünftigen Positionen der Füße für jeden Zeitschritt,

- Feststellung des FOG, bei Erreichen von Schrittlänge und Schrittfrequenz Null für Prädiktion,

- Ausgabe von Feedback an den Nutzer,

- Erwarten der Reaktion des Nutzers.

Im Folgenden werden die einzelnen Elemente und ihre Funktion erklärt.

Als Display wird eine Anzeigeeinrichtung bezeichnet, auf der Farbbilder angezeigt werden können. Normalerweise sind solche Anzeigen auf der Frontseite des Smartphones angeordnet, möglich ist aber auch die Innenseite eines klappbaren Mobilphones oder die Einblendung der Farbbilder auf eine Brille.

Eine rückseitige Farbkamera wird als Einzelkamera oder als Doppelkamera ausgeführt, wobei letztere räumliche Farbbilder aufnehmen kann.

Zusätzlich zur Bestimmung von Schrittfrequenz und Schrittlänge für jeden Zeitschritt können auch die Schrittbreite und andere Metriken bestimmt werden, die sich aus den Rohdaten berechnen lassen.

Das Feedback kann sowohl Stimulation an den Nutzer sein, als auch dem Nutzer als Grundlage für eine rein nutzergeführte Bewegungsanalyse dienen.

Mit AR, die Abkürzung steht für "Augmented Reality" sind Anwendungen gemeint, bei denen ein bestehendes Farbbild oder ein Farbfilm mit künstlich erzeugten Bildelementen angereichert werden kann. Diese künstlichen Bildelemente ersetzen entweder Teile des Originalbildes oder des Originalfilms, oder sie überlagern sie in transparenter Weise. Damit sie den Objekten auf den Bildern oder Filmen zugeordnet werden können, müssen diese Objekte zuvor bestimmt und mit Koordinaten versehen werden. Diese Zuordnung wird als Kalibrierung bezeichnet.

Unter AR-Toolkit versteht man eine Software, die unter Verwendung verschiedener Sensoren wie Kamera und IMU sowie ggf. LIDAR fortlaufend die Position und Ausrichtung des AR-Devices im Raum bestimmt und die auf dem Display darzustellenden virtuellen Inhalte mit dem Kamerastream vereinigt, in der Art, dass die virtuellen Inhalte in der Umgebung verankert zu sein scheinen.

Die Position des Gerätes im dreidimensionalen Raum und die Geometrie der Umgebung werden üblicherweise mittels einer IMU zur Verfügung gestellt. Eine IMU ist eine räumliche Kombination mehrerer Inertialsensoren wie Beschleunigungssensoren und Drehratensensoren. Sie stellt die sensorische Messeinheit eines Trägheitsnavigationssystems dar. In Smartphones dienen sie zur Bewegungsdetektion zur Bildstabilisation des Kamerasystems. Zur Erfassung der sechs möglichen kinematischen Freiheitsgrade verfügt eine IMU über drei jeweils aufeinander orthogonal stehende Beschleunigungssensoren, Accelerometer für, die Erfassung der translatorischen Bewegung in x- bzw. y- bzw. z-Achse und drei orthogonal zueinander angebrachten Drehratensensoren für die Erfassung rotierender Bewegungen in x- bzw. y- bzw. z-Achse. Eine inertiale Messeinheit liefert als Messwerte drei lineare Beschleunigungswerte für die translatorische Bewegung und drei Winkelgeschwindigkeiten für die Drehraten. Als weiterer Sensor kann ein Magnetometer vorgesehen sein. Zur Positionsbestimmung sind oft auch IPS als Gebäudeinternes und GPS als Globales Positionsbestimmungssystem nützlich und verfügbar.

In weiteren Ausgestaltungen sind vorgesehen ein LIDAR-Scanner, eine zusätzliche frontseitige Kamera, eine sogenannte "Selfiekamera", sowie die Ausstattung der rückseitigen Kamera mit guter Lichtempfindlichkeit und ohne Infrarotfilterung. LIDAR ist dabei eine Abkürzung für Light detection and ranging oder Light imaging, detection and ranging und ist eine dem Radar verwandte Methode zur optischen Abstands- und Geschwindigkeitsmessung als eine Form des dreidimensionalen Laserscanning. Statt der Radiowellen wie beim Radar werden Laserstrahlen verwendet. Weiterhin können auch mehrere rückseitige Kameras vorgesehen werden, von denen eine eine Weitwinkelkamera sein kann, mit der sowohl die Füße als auch das zu begehende Gelände gleichzeitig erfasst werden kann. Auch ist es möglich eine Kamera nach vorne und eine auf die Füße auszurichten oder mehrere Kameras zur besseren Parallaxe einzusetzen oder eine stirnseitige Kamera vorzusehen.

In einem ersten Schritt werden die Position und die Ausrichtung des Smartphones selbst bestimmt. Hierzu muss der Patient das Smartphone so halten, als ob er eine Nachricht lesen würde und die Kamera aktivieren. Sofern ein AR-Toolkit zur Verfügung gestellt werden kann, sind die Positionsdaten des Smartphones aus der Schnittstelle zum AR-Toolkit verfügbar, üblicherweise werden diese etwa 60 mal pro Sekunde zwischengespeichert, abgerufen und verarbeitet.

Hierbei ist wichtig, dass die Füße des Patienten von der rückseitigen Kamera des Smartphones erfasst werden können. Sobald Position und Ausrichtung des Smartphones erkannt sind, wird daher in einem zweiten Schritt geprüft, ob die Ausrichtung der Kamera und das Sichtfeld einen Blick auf die Füße des Nutzers, also des Patienten zulassen.

Falls nicht, wird der Patient aufgefordert, dass Smartphone so zu halten, dass er durch den Kamerabildschirm seine Füße sehen kann. Falls ja, eventuell nach Korrektur der Haltung des Smartphones, wird der Patient aufgefordert zu bestätigen, dass es sich um seine eigenen Füße handelt, die er sieht, und nicht etwa die einer helfenden oder zufällig anwesenden Person.

Im nächsten Schritt erfolgt eine Mustererkennung mittels eines bekannten Verfahrens künstlicher Intelligenz zur Erkennung der Füße und deren genauer Position. Zu diesem Zweck ist es hilfreich, wenn der Patient zuvor ein Kamerafoto seiner Füße bzw. der Schuhe macht, damit die künstliche Intelligenz anhand bekannter Algorithmen die Erkennung der Füße lernt und deren Position aus den Positionsdaten des Smartphones lernen und errechnen kann, wenn sich die Füße bewegen. Vor allem kann dann auch erkannt werden, ob sich die Füße überhaupt bewegen. Ebenfalls hilfreich ist es, wenn der Patient von allen Schuhe oder Socken, die er im Alltag trägt, solche Fotos auf einer Foto-Datenbank anfertigt und mit sich führt.

Ein mittels eines eigenen Datensatzes trainiertes künstliches neuronales Netz, im Folgenden als "NN" bezeichnet und auch als "ein deep convolutional neural network" bekannt, hat sich als zuverlässige Möglichkeit bewährt, Positionen von Objekten auf Bildern zu bestimmen. Das NN macht dabei durch die Vielfalt des annotierten Datensatz einige Verallgemeinerungen, durch das es ihm möglich ist, Füße in vielen Formen und Farben zu erkennen. Entsprechend des Alltags des Patienten können durch ein solches NN nackte Füße, Füße mit Socken und Füße in Schuhen verwendet und erkannt werden.

Der Datensatz des trainierten NNs besteht aus Farb-Bildern von Füßen, denen sogenannte Bounding-Boxes, also kleinste Rechtecke, die den Fuß vollständig umschließen, zugeordnet sind. Dementsprechend sagt das NN auch die Koordinaten von rechteckigen Boxen voraus, wenn man ihm Bilder als Eingabe zu Verfügung stellt.

Sofern die Koordinaten der Umgebung, also auch dem Boden, auf dem die Füße stehen, bekannt sind, werden die Höhenkoordinaten des Bodens in den Datensatz übernommen, alternativ werden die Füße als Höhenkoordinate genutzt oder die Entfernung von Kamera und deren Positionsdaten werden mit der erkannten Größe der Füße abgeglichen und die Höhenkoordinaten werden hieraus ermittelt.

Es ist auch möglich, diese Arten der Erkennung der Höhenkoordinaten mittels eines Kalmanfilters miteinander zu verbinden und so die Genauigkeit und Zuverlässigkeit zu verbessern.

Im Idealfall besitzt das Smartphone einen LIDAR-Scanner, mit dem das Sichtfeld der Kamera abgetastet wird. Der LIDAR-Scanner arbeitet mit einem Laserstrahl, der reflektiert wird, und aus dessen Laufzeit auf die jeweilige Entfernung geschlossen wird. Da das gesamte Sichtfeld gescannt wird, ergeben sich auch die Höhenkoordinaten des Bodens und die Koordinaten der Füße.

Aus den Koordinaten der Füße wird den Bounding-Boxen die Position des rechten und des linken Fußes zugeordnet. Diese Positionsbestimmung wird für jeden Zeitschritt durchgeführt.

Neben den Koordinaten gibt das NN auch Auskunft über die "Sicherheit" der Erkennung als Maß für die Aktivierung des Netzes durch das Eingabe-Bild. Erkennungen mit niedriger Sicherheit werden verworfen.

Somit gibt das NN als Ausgabe für jeden Zeitschritt entweder keine Auskunft, etwa da die Sicherheit der Erkennung zu niedrig ist, oder es liefert Koordinaten für eine oder mehrere Bounding-Boxen.

Außer der Höhenkoordinate des Bodens, auf dem die Füße bzw. die Bounding-Boxen sich gerade befinden, interessiert in einer Ausgestaltung der Erfindung auch der Boden davor, damit sichergestellt ist, dass ein Schritt nach vorn überhaupt physisch möglich und sinnvoll ist.

So kann es sich bei dem Boden auch um unebenes Gelände, um eine Treppe, eine Rolltreppe, ein Laufband oder einen Aufzug handeln. Das bedeutet, dass der Boden ansteigend oder abfallend sein oder sich bewegen kann. Zur Erfassung der Bodenstruktur des Bodens vor den Füßen wird ebenfalls die Kamera und, wenn möglich, der LIDAR-Scanner genutzt. Wird festgestellt, dass ein Schritt nach vorn problematisch sein dürfte oder könnte, wird von einer FOG-Hilfe sofort abgesehen. Problematisch können auch Wasserflächen sein, da ein LIDAR bei Wasserflächen und Wassertropfen an seine Grenzen stößt.

Alternativ zur Bestimmung der Bounding-Box für jeden Zeitschritt mithilfe des NNs wird vorgesehen, nur an einigen wenigen Zeitschritten eine NN-Inferenz durchzuführen und an den verbleibenden Zeitschritten nur eine Verschiebung der betreffenden Pixelgruppen im Bild zu messen. Hierzu werden etablierte Optical-Flow-Algorithmen verwendet.

Füße sind im Vergleich zum Untergrund leicht erhaben und lassen sich mit einem LIDAR-Scanner gut aufspüren und erkennen. Steht also zusätzlich zur Farbkamera auch ein LIDAR-Scanner zur Verfügung, lassen sich die Tiefeninformationen des LIDAR-Scanners ebenfalls als Bilder in ein vorher eigens dafür trainiertes NN einspeisen, das wiederum die Genauigkeit der Detektion verbessert.

Im nächsten Schritt werden dem AR-Toolkit die Koordinaten der Bounding-Box übergeben. Bei Kenntnis der Geometrie des Raumes weist das AR-Toolkit jedem Punkt der zweidimensionalen Farb-Kamera-Aufnahmen mittels des Raycast-Algorithmus einen Punkt im dreidimensionalen Raum zu. Durch dieses Vorgehen wird jedem Zentrum einer Bounding-Box eine dreidimensionale Koordinate zugewiesen.

Das Koordinaten-System wird am Anfang dieses Prozesses vom AR-Toolkit gewählt und im Folgenden so weit wie möglich und sinnvoll mit der Umwelt konsistent gehalten. Hierbei entstehen zwangsläufig Ungenauigkeiten aufgrund von Messfehlern, die ein Datenrauschen bewirken, und die das AR-Toolkit fortlaufend versucht zu verringern. Produkt der Transformationen ist somit eine Serie von 3D-Koordinaten über die Zeit, die die verrauschte und möglicherweise fehldetektierte Trajektorie/Raumbahn der Füße darstellt.

Im nächsten Verarbeitungsschritt werden die Detektionen der Füße klassifiziert, und zwar nach den drei möglichen Ergebnissen:

    1. kein Fuß (falsch-positiv),
    2. linker Fuß,
    3. rechter Fuß.

Um dies durchzuführen, stehen drei Alternativen zur Auswahl zur Verfügung, die auch kombiniert angewendet werden können, wenn die Rechenkapazität dies in Echtzeit zulässt.

Technisch wird die erste Alternative dadurch bewirkt, dass das Display in einen rechten und linken Teil geteilt wird. Der horizontale Wert der Grenze zwischen beiden Teilen ist variabel gehalten und anpassbar. Sobald in einem Zeitschritt zwei Detektionen erfasst werden, wird angenommen, dass es sich um den linken und den rechten Fuß handelt. Der Mittelwert beider horizontaler Komponenten der beiden Füße im Koordinatensystem des Ausgabebildschirms bildet den neuen Grenzwert zur Unterscheidung zwischen rechts und links. Dabei wird selbstverständlich davon ausgegangen, dass sich die beiden Füße nebeneinander befinden und kein Teil des einen Fußes über dem anderen Fuß ist.

Die zweite Alternative wird herangezogen werden, indem man mit 3D-Koordinaten statt mit Bildschirmkoordinaten rechnet: Der Schwerpunkt der beiden gleichzeitigen Detektionen bildet den Stützvektor für die Grenze zwischen linker und rechter Seite. Der Normalen-Vektor der Ebene auf der der Patient sich bewegt und der Geschwindigkeitsvektor des Gerätes spannen mit dem Stützvektor eine Ebene auf, die die Grenze zwischen linker und rechter Seite darstellt.

Die dritte Alternative ist die Verwendung eines NNs wie z.B. einem rekurrenten Netz wie dem "long-short-term memory"-Netz, welches einzelne Zeitschritte ganzer Zeitserien klassifizieren kann. Das Netzwerk wird trainiert mit von Menschen annotierten Serien von Koordinaten, denen die jeweilige Klassifikation zugewiesen wurde. Auf der Grundlage einer neuen Serie von Koordinaten kann das trainierte NN entscheiden, ob es sich um keinen Fuß, einen linken oder einen rechten Fuß handelt.

Im darauffolgenden Schritt wird eine Verifikation der erkannten Füße durchgeführt und es wird das Messrauschen verringert. Dies beides erfolgt mittels der Methode "Particle Filter" mit Monte-Carlo-Simulation, um auch falsch-positive Klassifikationen der Füße auszuschließen. Alternativ kann auch ein "erweiterter Kalman-Filter" und ein einfacher "function fit" mit einem "Non-linear least squares"-Algorithmus zur Modellierung des Ganges verwendet werden.

Die bisher erzeugten Rohdaten über die Positionen der Füße und des Gerätes sind mit Messrauschen behaftet und es wird kein Wissen über die Art der Kinematik verwendet. Die hier beschriebene Methode zur Filterung der Daten soll gewisse Annahmen über die Form der Bewegung integrieren. Die Bewegung eines einzelnen Fußes lässt sich in Näherung durch eine Überlagerung von trigonometrischen Funktionen beschreiben, wenn man in das mitbewegte Koordinatensystem des menschlichen Körpers transformiert.

$$x(t) = \sum_{i=0}^{N} a_i \, \sin\!\left(\omega_i t + \phi_{0,i}\right)$$

wobei $x(t)$ die Position des Fußes (1D), $a_i$ die Amplitude, $\omega_i$ die Winkel-Schrittfrequenz und $\phi_{0,i}$ die Anfangsphase ist. Die Modellfunktion muss dabei nicht auf die Superposition von Sinus-Funktionen beschränkt sein, auch andere geeignete Funktionen können verwendet werden.

Die Bewegung des Körperschwerpunkts wird modelliert als System mit gleichförmiger Geschwindigkeit und Beschleunigung. Näherungsweise bildet es dabei die Bewegung des Gerätes als die Bewegung des Körperschwerpunktes ab.

Diese Modellannahmen werden in einem Particle-Filter implementiert. Die Positionen der Füße sind nach dem Verständnis des Modells eine Funktion von Schrittfrequenz und Amplitude proportional zur Schrittlänge, aufgetragen über die Zeit. Der Kalman-Filter macht hierbei Abschätzungen zur Position und Geschwindigkeit des menschlichen Körpers und der Füße unter der Berücksichtigung des unumgänglichen Mess- und Prozessrauschens, charakterisiert durch eine Gauss-Verteilung mit der Standartabweichung $\sigma$. Zunächst filtert der Particle-Filter die Positionen des Gerätes, reduziert das Rauschen und berechnet die Geschwindigkeit. Mit der Kenntnis der Geschwindigkeit des Gerätes transformiert der Filter in das Schwerpunktssystem des Körpers und bestimmt die Schrittfrequenz und Amplitude der Gehbewegung auf Grundlage der gemessenen Fußpositionen.

Auf diese Art und Weise werden mehrere Probleme zugleich gelöst:

    1. Das Messrauschen der Fuß-Positionen wird reduziert, es wird eine glattere Trajektorie errechnet.

    2. Es werden Parameter wie Schrittfrequenz und Schrittlänge bestimmt

3. Das System macht Vorhersagen über die zukünftigen Positionen der Füße, im Folgenden als Prädiktionen bezeichnet. Das ist besonders erstrebenswert, um ein flüssiges Nutzer-Feedback zu gewährleisten. Wenn das System weiß, wann welcher Fuß gesetzt wird, können bereits nächste Schritte zur Anpassung der AR-Umgebung geplant werden, und der Nutzer hat Gefühl, mit einem sehr reaktionsschnellen System zu arbeiten, da das System schon reagiert, bevor überhaupt schon ein Fuß gesetzt wurde.

4. Das System kann die Trajektorie der Füße auch in kurzen Phasen, in denen die Kamera keinen visuellen Kontakt zu den Füßen hat, modellieren.

5. Der Filter kann falsch-positive Detektionen ausschließen, wenn die Detektion etwa um einige zuvor festgelegte Standardabweichungen von der Prädiktion des Filters abweicht.

6. Arrhythmien des Ganges können leicht erkannt werden, wenn sich systemische Abweichungen von der Prädiktion ergeben.

Die Höhe des Gerätes über der Ebene, in der sich der Nutzer bewegt, unterliegt ebenfalls zyklischen Schwankungen synchron zur Gehbewegung. Es kann optional zur Modellierung in den Filter eingespeist werden.

In einer weiteren Ausgestaltung der Erfindung wird die Bewegung der Füße modelliert durch ein rekurrentes NN, wie ein "long-short term memory"-Netzwerk. Das NN wird trainiert auf die Vorhersage von Fußpositionen mithilfe von Daten von einem etablierten Motion Capturing System, das eine kontinuierliche Erfassung der Füße erlaubt. Eingabedaten für das Netzwerk sind die Bewegungsdaten des Smartphones und vergangene Detektionen, Ausgabedaten sind die vorhergesagten Fußpositionen, gemessen von dem etablierten Motion Capturing System. Das erfüllt zwei Zwecke:
Zum einen werden Lücken in der Serie von Detektoren gefüllt, wenn der Fuß hinter dem Körper verschwindet und die Kamera, die Füße nicht mehr erfassen kann. Fallen Detektionen durch Verdeckung der Kamera-Sichtachse aus, kann das NN die Lücken mit Prädiktionen füllen, bis wieder Detektionen verfügbar sind.

Zum anderen werden die Vorhersagen des NNs assoziiert mit den in Echtzeit eingehenden Detektionen, um zu entscheiden, ob es um einen rechten, einen linken Schritt oder um eine Fehldetektion handelt. Zur Entscheidung, zu welcher Klasse eine neue Detektion gehört kann ein Vergleich der euklidischen Distanz zwischen Prädiktion und neuer Detektion herangezogen werden, oder ein weiteres NN erlernt die Entscheidung anhand annotierter Daten.

Zur genaueren Bestimmung der Positionen, an denen ein Fuß zum Stehen kommt, und sich damit relativ zum Weltkoordinatensystem nicht bewegt, wird über einen Mean-Shift-Algorithmus bestimmt. Der Algorithmus berechnet den Gauss-gewichteten Schwerpunkt aller Detektions-Koordinaten an einer gewählten Position. Der Verschiebungsvektor

$$\mathbf{m}_h(\mathbf{x}) = \frac{\sum_{i=1}^{n} \mathbf{x}_i g\left(\left\|\frac{\mathbf{x}-\mathbf{x}_i}{h}\right\|^2\right)}{\sum_{i=1}^{n} g\left(\left\|\frac{\mathbf{x}-\mathbf{x}_i}{h}\right\|^2\right)} - \mathbf{x}$$

zeigt zum Punkt der höchsten Dichte im gescreenten Bereich, abhängig von der Breite der Gauss-Gewichtung. Hier bei sind $x_i$ die gemessenen Koordinaten und $g(0, \sigma)$ eine Gauss-Funktion mit Mittelwert 0 und Breite $\sigma$. Ausgehend von einem Startpunkt wird iterativ der m-Vektor berechnet, bis dieser hinreichend klein ist. Der Punkt der Konvergenz bzw. das aktuelle Maximum der Dichteverteilung ist dann erreicht.

Bei einer Echtzeitanwendung liegt die gesamte Verteilung der Messungen zu diesem Zeitpunkt noch nicht vor. Ist der Fuß noch in Bewegung oder im Abbremsvorgang, wird hilfsweise für jede Ausführung des Mean-Shift-Algorithmus am aktuellen Zeitpunkt ein neues, vorübergehendes Maximum gebildet. Steht der Fuß, konvergieren alle Ausführung des Mean-Shift-Algorithmus für beliebige Startpunkte auf einen einzelnen Punkt, da sich an dem Punkt die Detektionen häufen. Sobald der Algorithmus für verschiedene Startpunkte mehrfach in einem Endpunkt konvergiert, wird angenommen, dass es sich um einen Stillstand des Fußes handelt.

Das beschriebene Verfahren bringt gleichzeitig den Vorteil, den geschätzten Mittelwert der Detektionen zu erhalten, entsprechend der Annahme, dass die Messungen mit einem Gauss'schen Fehler behaftet sind.

Der Abstand zwischen den Punkten, an denen der Fuß zum Stehen kommt, ist proportional zur Schrittlänge. Mit dem Skalarprodukt aus dem Verschiebungsvektor zwischen zwei Fuß-Stillstand-Positionen (linker und rechter Fuß) $\vec{s}$ und dem mittleren Bewegungsvektor des Gerätes $\vec{v}$, lässt sich die Schrittlänge L einfach errechnen. Die so errechneten Parameter dienen auch der Anpassung der Ausgabe für den Nutzer. Die Ausgabe kann verschiedene Modalitäten umfassen.

Zur Auswahl stehen drei optionale Arten, dem Patienten ein Feedback zur Verfügung zu stellen, je nach Präferenz.

1. Haptisches Feedback mittels eines Vibrationsmotors, etwa durch kurze rhythmische Vibrationen,
2. akustisches Feedback über einen Lautsprecher, etwa durch kurze, rhythmische Töne oder Laute,
3. visuelles Feedback über einen Bildschirm, etwa den eines handelsüblichen Smartphones oder ein "head-mounted-display", das am Kopf des Nutzers befestigt wird.

Das Display kann ein gewöhnliches intransparentes Display sein, das sowohl im ein- als auch ausgeschalteten Zustand kein Licht von außen durchlässt. Üblicherweise sind solche Systeme mit Akkomodationslinsen versehen, um eine angemessene virtuelle "Entfernung" des angezeigten Inhaltes für den Nutzer einzustellen.

Ebenfalls möglich ist ein transparentes Display, das Lichtstrahlen von außen zum Auge des Nutzers durchlässt und additiv visuelle Informationen zum Gesichtsfeld des Nutzers hinzufügt. Letztere Option ist für die hier vorgestellte Realisierung besonders vorteilhaft.

In einer Ausgestaltung der Erfindung wird vorgesehen, dass abhängig von der gemessenen Schrittfrequenz auch die Frequenz der Vibrationen und Tonpulse angeglichen werden, um den Nutzer in seinem Tempo zu stimulieren.

Die Schrittlänge und die aktuelle Fußposition werden verwendet, um die Inhalte auf dem Display des Nutzers anzupassen. Da das AR-Toolkit fortlaufend die Geometrie des Raumes und die Position des Nutzers erfasst, kann die Software geometrische Objekte wie Linien und Quader zielgerichtet im Sichtfeld des Nutzers einblenden. Die Objekte lassen sich in regelmäßigen Mustern anordnen, z.B. lassen sich Linien in regelmäßigen Abständen auf dem Fußboden vor dem Nutzer darstellen. Die Abstände zwischen den Linien werden bestimmt durch die Schrittlänge. Schafft es der Nutzer nicht, die nächste vor ihm liegende Linie zu übertreten, wird die Linie nach hinten zum Fuß des Nutzers verschoben und die Abstände zu den nächsten Linien werden entsprechend der aktuellen Schrittlänge angepasst.

In weiteren Ausgestaltungen können auch mehrere rückseitige Kameras vorgesehen werden, von denen eine eine Weitwinkelkamera sein kann, mit der sowohl die Füße als auch das zu begehende Gelände gleichzeitig erfasst werden kann. Auch ist es möglich eine Kamera nach vorne und eine auf die Füße auszurichten oder mehrere Kameras zur besseren Parallaxe einzusetzen.

In einer vorteilhaften Ausgestaltung wird vorgesehen, einen Schrittzähler oder ein Belohnungssystem im Blickfeld des Nutzers einzublenden, das jedes Mal ausgelöst wird, wenn der Nutzer sein Ziel erreicht. Ein Ziel kann sein, eine gewisse Strecke zu laufen, eine Linie oder ein virtuelles Hindernis zu überqueren, eine gewisse Laufgeschwindigkeit oder eine virtuell markierte Position zu erreichen. Das System kann somit auch vorteilhaft zum Training eingesetzt werden.

Alle gemessenen Parameter werden verwendet, um den Zustand des Bewegungsapparates des Nutzers zu erfassen. Erfährt der Nutzer eine Episode von FOG, ist es sinnvoll, den Nutzer mit multimodalen Cues zu stimulieren. Die Frequenzen der akustischen und haptischen Cues sind abhängig von der gemessenen Schrittfrequenz und die Abstände der visuellen Cues sind abhängig von der Schrittlänge. Cueing sollte nur dann angewendet werden, wenn es wirklich benötigt wird, um ein Abnutzen der Cues zu vermeiden. Die verschiedenen Stimuli können vom System auch vorteilhaft getestet werden, da nicht jeder Nutzer auf die Cues in gleicher Weise reagiert. Verschiedene Stimuli können sein: verschiedenfarbige Linie auf dem Boden, ein Quader oder Block auf dem Boden, Simulation eines rollenden Gegenstandes in Richtung der intendierten Gehbewegung, um dem Patienten ein Gefühl von "Fluss" zu geben, verschiedene Töne/Klicklaute in bestimmten Frequenzen, Vibrationen, oder Klingeltönen. Die verschiedenen Stimuli werden nacheinander dem Patienten dargeboten und das System misst, wie gut der Patient auf die verschiedenen Stimuli reagiert, indem das System schaut, ob sich z.B. Schrittlänge und Schrittfrequenz verbessert haben. Die automatische Durchtestung und fortlaufende Anpassung der Stimuli während der Benutzung kann durch ein lernfähiges KI-System erreicht werden.

Optional wird dem Nutzer ein Trainingsplan eingespielt. Der Trainingsplan sieht eine festgelegte Abfolge von Stimuli aus dem genannten Repertoire vor, die dem Nutzer nacheinander dargeboten werden. Werden visuelle Stimuli dargeboten, ist der Patient aufgefordert, diese Landmarken wie etwa Linien in der AR abzulaufen. Werden Stimuli haptischer oder akustischer Natur eingespielt, ist der Nutzer aufgefordert, seine Schrittfrequenz, der der Stimuli anzupassen. Der Trainingsplan wird fest in die Applikation einprogrammiert oder über das Internet eingespielt. Eingespielte Trainingspläne werden von medizinischem Fachpersonal erstellt. Die Applikation überwacht die Ausmaße der Ausführung und des Trainingserfolgs des Trainingsplans. Metriken zur Quantifizierung der Ausführung sind die Mittelwerte der räumlichen Abstände der Füße von den vom Trainingsplan vorgegebenen Landmarken und die Zeitintervalle zwischen der Erreichung der einzelnen Landmarken, sowie der Gesamtzeitraum der Trainingseinheit. Die Metriken werden dem Fachpersonal über das Internet zur Verfügung gestellt.

In einer vorteilhaften Ausgestaltung wird vorgesehen, dass zum Erkennen von FOG ein aus Schrittlängen und Schrittfrequenz gebildeter Parameter das errechnete Ergebnis mit einem zuvor festgelegten Schwellenwert vergleicht.

Die Erfindung kann ebenfalls genutzt werden zur Überwachung des motorischen Zustandes des Patienten im häuslichen Umfeld. Der Patient wird angewiesen, eine definierte Wegstrecke zu laufen. Das AR-Gerät erfasst die Bewegung der Füße und erstellt eine statistische Auswertung wichtiger Metriken, wie Schrittlänge, Schrittbreite, Kadenz, Schrittgeschwindigkeit und Seitenexkursion. Ebenfalls lassen sich in der statistischen Auswertung Abweichungen in der Seitensymmetrie bei der Bewegung des rechten und linken Fußes finden. Für Drehungen die der Patient vollführt, lassen sich die Anzahl der benötigten Schritte zählen. Die automatische Auswertung wird dem Fachpersonal über das Internet zur Verfügung gestellt.

Die Erfindung wird im Folgenden anhand von Beispielen näher erläutert, wobei diese Beispiele aber nicht limitierend für den Umfang der Erfindung sind.

In Beispiel 1 wird eine Realisierung mittels eines Smartphones mit einer App gezeigt. Die zur Verfügung stehenden Sensoren dieser Realisierung sind ein Accelerometer, ein Gyroskop, ein Magnetometer und eine Farb-Kamera. Als Ausgabe stehen zur Verfügung ein Display, ein Vibrationsmotor und ein Lautsprecher. Der Nutzer hält das Smartphone in stehender Position in der Hand. Das Smartphone ist so ausgerichtet, dass die Kamera in Richtung des Bodens zeigt und die Füße oder mindestens ein Fuß im Sichtfeld der Kamera liegen. Der integrierte Prozessor führt den oben beschriebenen Algorithmus aus. Passen gemessene Schrittlänge und Schrittfrequenz mit der gemessenen Gesamtgeschwindigkeit zusammen, wird die Ausgabe für den Nutzer gestartet. Der Nutzer erfährt zyklisches Feedback in den oben genannten Modalitäten.

In Beispiel 2 wird eine Realisierung mittels einer Augmented-Reality-Brille mit transparentem "Head-mounted Display" gezeigt. Solche Brillen sind in unauffälligen Ausführungen erhältlich. Die zur Verfügung stehenden Sensoren dieser Realisierung sind ein Accelerometer, ein Gyroskop, ein Magnetometer und eine Farb-Kamera. Als Ausgabe stehen zur Verfügung ein Display und ein Lautsprecher. Das Display ist direkt vor einem oder beiden Augen befestigt. Durch die transparenten Eigenschaften des Displays kann der Nutzer die Umgebung hinter dem Display sehen. Der integrierte Prozessor führt den oben beschriebenen Algorithmus aus. In dieser Realisierung kann die Notwendigkeit zum Cueing durch einen besonders einfachen Mechanismus erkannt werden: Sobald der Patient den Kopf abwärts bewegt und (länger) auf seine Füße schaut, beginnt das System mit den Berechnungen für das Cueing. Die Kopfabwärtsbewegung kann durch die Positionssensoren (z.B. Gyroskop) erkannt werden. Denkbar wäre auch die Nutzung eines Systems zur Verfolgung der Blickrichtung der Pupillen, das überwacht, wann der Nutzer auf seine Füße schaut. Passen gemessene Schrittlänge und Schrittfrequenz mit der gemessenen Gesamtgeschwindigkeit zusammen, wird die Ausgabe für den Nutzer gestartet. Der Nutzer erfährt zyklisches Feedback in den oben genannten Modalitäten.

In Beispiel 3 wird eine Realisierung mittels eines Smartphones, welches über einen rückseitigen LIDAR verfügt und mit einer App ausgestattet ist, gezeigt. Die zur Verfügung stehenden Sensoren dieser Realisierung sind über den LIDAR hinaus ein Accelerometer, ein Gyroskop, ein Magnetometer, eine rückseitige Farb-Kamera und eine frontseitige Selfie-Kamera. Ferner verfügt das System über einen IPS- oder GPS-Empfang und eine Sendeeinheit. Als Ausgabe stehen zur Verfügung ein Display, ein Vibrationsmotor und ein Lautsprecher. Der Nutzer hält das Smartphone in stehender Position in der Hand. Das Smartphone ist so ausgerichtet, dass die Kamera in Richtung des Bodens zeigt und die Füße oder mindestens ein Fuß im Sichtfeld der Kamera liegen, was dem Nutzer über den frontseitigen Bildschirm angezeigt wird. Die frontseitige Kamera prüft dabei, ob sie das Gesicht des Nutzers sehen und ihn auch erkennen kann.

Ein solcher Sichtkontakt ist nur in gewissen Zeitabständen erforderlich und sinnvoll, da der Nutzer auch seine Umgebung beachten muss und nicht die ganze Zeit auf sein Smartphone starren darf. Die frontseitige Kamera überprüft dies, wobei sie die Pupillen des Nutzers mit dem üblichen Rotabgleich für angestrahlte Pupillen und deren Ausrichtung analysiert.

Falls weder die rückseitige Kamera noch der rückseitige LIDAR die Füße erkennen kann, oder wenn die frontseitige Kamera keinen auf sie gerichteten Blick des Nutzers erkennt, fordert das Smartphone den Nutzer zu einer Korrektur der Ausrichtung des Smartphones auf. Reagiert der Nutzer hierauf nicht, alarmiert das Smartphone über seine Sendeeinrichtung helfendes Personal, welches über die Telefoneigenschaft Kontakt zum Nutzer aufnehmen kann. Falls dies nicht möglich ist, bewirkt der Alarm eine Suchaktion mit Unterstützung des IPS- oder GPS-Senders.

Falls die Ausrichtung des Smartphones korrekt ist, wird mittels IPS oder GPS eine Positionsbestimmung vorgenommen und mittels des LIDAR wird geprüft, ob der einzuschlagende Weg des Nutzers gangbar ist. Dann beginnt derselbe Vorgang wie in Beispiel 1, wobei der LIDAR die Erkennung der Position der Füße unterstützt. Beim Abgleich der erkannten Positionsdaten sowie der Reduzierung des Messrauschens werden auch die Daten des LIDAR mitverwendet, was die Konvergenz bei der Berechnung und damit die Echtzeitfähigkeit bei Ausführung des erfindungsgemäßen Erkennungsverfahrens erheblich beschleunigt.

Passen gemessene Schrittlänge und Schrittfrequenz mit der gemessenen Gesamtgeschwindigkeit zusammen, wird die Ausgabe für den Nutzer gestartet. Der Nutzer erfährt zyklisches Feedback in den oben genannten Modalitäten, welches ergänzt wird durch Geländedaten wie etwa Hindernisse, Treppen, Geländer, Wände oder Straßenverkehr. Letzteres verhindert, dass der Nutzer zum Weitergehen animiert wird, wenn ein Stehenbleiben vernünftig ist und nicht als FOG gewertet werden darf, beispielsweise an einer roten Fußgängerampel.

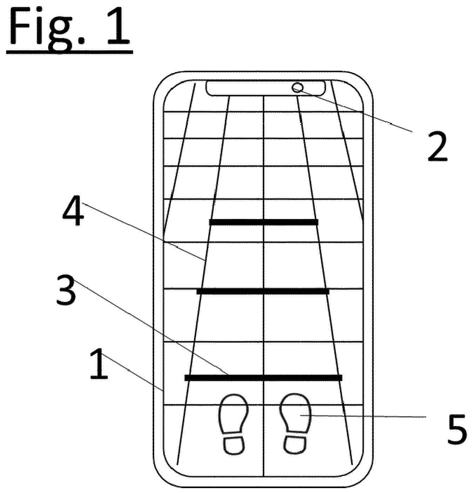

Fig. 1 zeigt ein Display 1 eines Smartphones und einer Frontkamera 2, und Linien 3, die dem Nutzer die nächsten Schritte vorgeben. Gleichzeitig werden dem Nutzer der Fußboden 4, hier als gefliester Boden dargestellt, sowie die Position seiner Füße 5 zur Orientierung angezeigt. Damit kann der Nutzer auf einfachste Weise stimuliert werden, wenn sich aus den Bewegungsdaten ein FOG zeigt.

**Patentansprüche**

1. Verfahren zur Unterstützung einer Gehbewegung einer Person mit einer Bewegungsstörung, mittels eines Smartphones, aufweisend

• ein Display (1),
• eine rückseitige Farbkamera, die für Augmented-Reality-(AR-) Anwendungen kalibriert ist, und Sensoren für die Position des Gerätes im dreidimensionalen Raum und die Geometrie der Umgebung integriert hat,
• eine Schnittstelle für ein AR-Toolkit sowie ein AR-Toolkit,
• Sensoren für Sensordaten aus einer Inertial Measurement Unit (IMU),

umfassend die folgenden Schritte:

• anfängliche Bestimmung von Position und Ausrichtung des Smartphones,
• anfängliche Prüfung, ob die Ausrichtung der Kamera und das Sichtfeld einen Blick auf die Füße (5) des Nutzers zulässt,
• Mustererkennung zur Erkennung der Füße (5) und deren genauer Position für jeden Zeitschritt,
• Definition der Bounding-Boxen für die Füße (5) und deren Koordinaten einschließlich der Höhenkoordinaten für jeden Zeitschritt,
• Zuordnung der Position der Bounding-Boxen zum rechten und linken Fuß für jeden Zeitschritt,
• Analyse der Zuverlässigkeit der Positionsdaten für jeden Zeitschritt,
• Übergabe der Bildschirmkoordinaten der Bounding-Boxen an das AR-Toolkit, für jeden Zeitschritt,
• Anfertigung eines Abbildes des Raumes durch die rückseitige Kamera und Zuordnung jedes Bildpunktes einem Punkt des abgebildeten Raumes durch das AR-Toolkit für jeden Zeitschritt,
• Anfertigung einer zeitlichen Serie von Raumpunkten der Bounding-Boxen für jeden Zeitschritt,
• Klassifikation der Detektion der Füße (5) für jeden Zeitschritt,
• Reduktion des Messrauschens für jeden Zeitschritt,
• Bestimmung von Schrittfrequenz und Schrittlänge für jeden Zeitschritt,
• Generierung von Prädiktionen über die zukünftigen Positionen der Füße für jeden Zeitschritt,
• Feststellung des "Freezing of Gait", bei Erreichen von Schrittlänge und Schrittfrequenz Null für Prädiktion,
• Ausgabe von Feedback an den Nutzer,
• Erwarten der Reaktion des Nutzers.

2. Verfahren nach Anspruch 1, zusätzlich aufweisend einen LIDAR, umfassend zusätzlich die folgenden Schritte mittels des LIDAR:

• Erkennen der Bounding-Boxes,
• Erkennung des zu beschreitenden Geländes,
• Zuordnung der Bilddaten der rückseitigen Kamera zu den Raumkoordinaten,
• Warnung an den Nutzer, falls der eingeschlagene Weg nicht gangbar ist.

3. Verfahren nach Anspruch 1, zusätzlich aufweisend eine frontseitige Kamera (2), umfassend zusätzlich die Schritte

• Prüfung, ob der Nutzer seinen Blick auf das Display richtet,
• Ausgabe eines akustischen oder haptischen Hinweises, dass er seinen Blick auf das Display richten möge, wenn er dies nicht tut, obwohl gerade ein optischer Hinweis erfolgt ist.

4. Verfahren nach Anspruch 1, wobei die rückseitige Kamera keinen Infrarotfilter aufweist, umfassend zusätzlich den Schritt

• Detektion der Füße aufgrund der Wärmestrahlung im Infrarotbereich.

5. Verfahren nach den Ansprüchen 1 bis 4, **dadurch gekennzeichnet, dass** die verschiedenen Erkennungsmethoden für die Position der Füße (5) sowie die Klassifikation und die Reduktion des Messrauschens mittels eines Particle-Filters miteinander abgeglichen werden und dieser Abgleich für die Prädiktion verwendet wird.

6. Verfahren nach den Ansprüchen 1 bis 4, **dadurch gekennzeichnet, dass** die verschiedenen Erkennungsmethoden für die Position und Bewegung der Füße (5) mittels eines ein rekurrenten neuronalen Netzes modelliert wird, wobei das NN auf die Vorhersage von Fußpositionen mithilfe von Daten von einem etablierten Motion Capturing System trainiert wird.

7. Verfahren nach einem der Ansprüche 1 bis 6, **dadurch gekennzeichnet, dass** die Prädiktion zur Projektion von visuellem Feedback in Form von Markierungen oder Linien (3) als Anzeige auf dem Display (1) verwendet wird.

8. Verfahren nach einem der Ansprüche 1 bis 7, **dadurch gekennzeichnet, dass** die Prädiktion mittels kurzer rhythmischer Vibrationen oder Töne unterstützt wird.

9. Vorrichtung zur Unterstützung einer Gehbewegung einer Person mit einer Bewegungsstörung, aufweisend

• ein Display (1),
• eine rückseitige Farbkamera, die für AR-Anwendungen kalibriert ist, und Sensoren für die Position des Gerätes im dreidimensionalen Raum und die Geometrie der Umgebung integriert hat,
• Sensoren für Sensordaten aus einer IMU,
• eine Schnittstelle für ein AR-Toolkit sowie ein AR-Toolkit,

**dadurch gekennzeichnet, dass**

die Vorrichtung ein darauf installiertes Programm aufweist, welches die folgenden Schritte auszuführen in der Lage ist:

• anfängliche Bestimmung von Position und Ausrichtung des Smartphones,
• anfängliche Prüfung, ob die Ausrichtung der Kamera und das Sichtfeld einen Blick auf die Füße (5) des Nutzers zulässt,
• Mustererkennung zur Erkennung der Füße (5) und deren genauer Position für jeden Zeitschritt,
• Definition der Bounding-Boxen für die Füße (5) und deren Koordinaten einschließlich der Höhenkoordinaten für jeden Zeitschritt,
• Zuordnung der Position der Bounding-Boxen zum rechten und linken Fuß für jeden Zeitschritt,
• Analyse der Zuverlässigkeit der Positionsdaten für jeden Zeitschritt,
• Übergabe der Bildschirmkoordinaten der Bounding-Boxen an das AR-Toolkit, für jeden Zeitschritt,
• Anfertigung eines Abbildes des Raumes durch die rückseitige Kamera und Zuordnung jedes Bildpunktes einem Punkt des abgebildeten Raumes durch das AR-Toolkit für jeden Zeitschritt,
• Anfertigung einer zeitlichen Serie von Raumpunkten der Bounding-Boxen für jeden Zeitschritt,
• Klassifikation der Detektion der Füße (5) für jeden Zeitschritt,
• Reduktion des Messrauschens für jeden Zeitschritt,
• Bestimmung von Schrittfrequenz und Schrittlänge für jeden Zeitschritt,
• Generierung von Prädiktionen über die zukünftigen Positionen der Füße für jeden Zeitschritt,
• Feststellung des "Freezing of Gait", bei Erreichen von Schrittlänge und Schrittfrequenz Null für Prädiktion,
• Ausgabe an den Nutzer,
• Erwarten der Reaktion des Nutzers.

10. Vorrichtung nach Anspruch 9, zusätzlich aufweisend einen rückseitigen LIDAR.

11. Vorrichtung nach Anspruch 9, zusätzlich aufweisend eine frontseitige Kamera (2).

12. Vorrichtung nach Anspruch 9, wobei die rückseitige Kamera keinen Infrarotfilter besitzt.

13. Vorrichtung nach Anspruch 9, zusätzlich aufweisend ein Head-mounted Display (1).

14. Vorrichtung nach Anspruch 9, zusätzlich aufweisend einen Lautsprecher für kurze, rhythmische Töne oder Laute.

15. Vorrichtung nach Anspruch 9, zusätzlich aufweisend einen Vibrationsmotor für kurze rhythmische Vibrationen

# Fig. 1

Europäisches Patentamt
European Patent Office
Office européen des brevets

# EUROPÄISCHER RECHERCHENBERICHT

**Nummer der Anmeldung**

EP 22 18 3460

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | DE 10 2014 215487 A1 (BOSCH GMBH ROBERT [DE]) 11. Februar 2016 (2016-02-11) * Abbildungen 1,3 * * Absatz [0037] – Absatz [0049] * * Absatz [0053] * * Absatz [0076] * ----- | 1-15 | INV. A61B5/11 G06V10/44 A61B5/00 |
| A | NOUREDANESH MINA ET AL: "Chasing Feet in the Wild: A Proposed Egocentric Motion-Aware Gait Assessment Tool", 23. Januar 2019 (2019-01-23), ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 176 – 192, XP047501284, ISBN: 978-3-319-10403-4 [gefunden am 2019-01-23] * Abbildungen 1-3 * * Seite 180, Zeile 1 – Zeile 9 * * Seite 182, Zeile 1 – Seite 183, Zeile 3 * ----- -/-- | 1,9 | RECHERCHIERTE SACHGEBIETE (IPC) A61B G06K G06V |

Der vorliegende Recherchenbericht wurde für alle Patentansprüche erstellt

| Recherchenort | Abschlußdatum der Recherche | Prüfer |
|---|---|---|
| Den Haag | 22. November 2022 | Oancea, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

**Nummer der Anmeldung**

**EP 22 18 3460**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | MAZILU SINZIANA SINZIANA MAZILU@IFE EE ETHZ CH ET AL: "GaitAssist a daily-life support and training system for parkinson's disease patients with freezing of gait", PROCEEDINGS OF THE SIGCHI CONFERENCE ON HUMAN FACTORS IN COMPUTING SYSTEMS, ACM, 2 PENN PLAZA, SUITE 701 NEW YORK NY 10121-0701 USA, 26. April 2014 (2014-04-26), Seiten 2531-2540, XP058617667, DOI: 10.1145/2556288.2557278 ISBN: 978-1-4503-2473-1 * Zusammenfassung * * Abbildung 3 * * Seite 2534, Spalte 2, Zeile 25 - Seite 2535, Spalte 1, Zeile 12 * ----- | 1,9 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 22. November 2022 | Oancea, A |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 22 18 3460

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-11-2022

| Im Recherchenbericht angeführtes Patentdokument | Datum der Veröffentlichung | Mitglied(er) der Patentfamilie | Datum der Veröffentlichung |
|---|---|---|---|
| DE 102014215487 A1 | 11-02-2016 | KEINE | |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014215487 A1

**In der Beschreibung aufgeführte Nicht-Patentliteratur**

- AI: GaitAssist: A Daily-Life Support and Training System for Parkinson's Disease Patients with Freezing of Gait. **SCHRIFT MAZILU, S.** One of a CHInd, Toronto, ON, Canada; Session Interactive Technologies for Rehabilitation. CHI, 2014, 2531-2540